# EUROPEAN PATENT APPLICATION

(11) **EP 2 088 205 A1**
(43) Date of publication of application: **12.08.2009**
(21) Application number: 08300084.4
(22) Date of filing: 11.02.2008
(51) Int. Cl.: C12Q 1/68

(54) **PSMB10: A diagnosis marker and therapeutic target of chronic rejection.**

(71) Applicant: INSTITUT NATIONAL DE LA SANTE ET DE LA RECHERCHE MEDICALE (INSERM), 75013 Paris (FR)
(72) Inventor: Brouard, Sophie, 44240 Suce Sur Erdre (FR); Giral, Magali, 44470 Carquefou (FR); Soulillou, Jean-Paul, 44000 Nantes (FR); Jovanovic, Vojislav, 44000 Nantes (FR); Ashton-Chess, Joanna, 44000 Nantes (FR)
(74) Representative: Warcoin, Jacques

(57) **Abstract**

The present invention relates to a method for diagnosing chronic graft rejection of a grafted organ in a subject from a biological sample of said subject, comprising: (a) determining in vitro an expression level value for PSMB10 in said subject biological sample, (b) comparing said value to at least one reference expression level value for PSMB10 in at least one reference sample, and (c) diagnosing if said subject is or not undergoing chronic rejection of said grafted organ. The invention also concerns a diagnostic kit or microarray for performing the method of the invention. The invention further concerns the medical use of proteasome inhibitors for treating chronic rejection.

## Description

### FIELD OF THE INVENTION

The present invention relates to a method for diagnosing chronic rejection of a grafted organ in a subject from a biological sample of said subject, comprising: (a) determining in vitro an expression level value for PSMB10 in said subject biological sample, (b) comparing said value to at least one reference expression level value for PSMB10 in at least one reference sample, and (c) diagnosing if said subject is or not undergoing chronic rejection of said grafted organ. The invention also concerns a diagnostic kit or microarray for performing the method of the invention. The invention further concerns the medical use of proteasome inhibitors for treating chronic rejection.

### BACKGROUND ART

More than 250 000 Europeans live with an organ transplant and 80 000 remain on transplant waiting lists. Increasing the life of a transplanted organ remains a major challenge in transplantation. Indeed, despite an increase in graft survival and a decrease in the risks of rejection, the leading cause of graft loss, chronic rejection, is only poorly influenced by currently used immunosuppressors.

Deciphering the mechanisms of such late graft loss would enable more personalized treatment strategies, but is hindered by the difficulty in assigning specific diagnoses.

In addition, chronic rejection is usually diagnosed on a histological basis, by studying a biopsy of the grafted organ. However, a biopsy is an invasive examination, which is not without danger for the grafted organ, and is thus usually not performed on grafted subjects that have stable biological parameters values. In addition, the variability of the diagnosis, due to the subjectivity of the analysis, is a drawback of the histological examination of biopsies.

Furthermore, in the case of many grafted organ, when the values of standard biological parameters allow for the diagnosis of chronic rejection, the rejection process is already in progress and, although it may in certain cases be stopped, the lesions that have been induced generally cannot be reversed. Moreover, to confirm the diagnosis, a biopsy of the grafted organ is usually performed, which is, as stated before, not without danger.

It would thus be very valuable to have a non-invasive method allowing diagnosing chronic rejection at the earlier steps of the rejection process, which would permit to adapt the immunosuppressive treatment and might in some cases prevent the chronic rejection process. Such a method should be sensitive enough to detect chronic rejection at its earlier steps. It should also preferably be non invasive, which would permit to perform control tests on a regular basis, without affecting the grafted subject's quality of life or endangering the survival of the graft.

Altogether, there is thus a need for a non invasive, simple, easy to perform and reliable method for diagnosing chronic graft rejection in a subject based on a biological sample of said subject.

The use of DNA microarrays to exhaustively screen the genome is an innovative approach in the field of transplantation.

Flechner et al (1) describe the comparison, using microarray technology, of the expression profiles of kidney biopsies or peripheral blood lymphocytes (PBLs) from normal kidneys donors, well-functioning kidney transplanted subjects, kidney transplanted subjects undergoing acute rejection and kidney transplanted subjects suffering from renal dysfunction without rejection. The authors identified distinct signatures for both biopsies and PBLs associated with each of the clinical states. However, no analysis of chronic rejection, which corresponds to distinct rejection mechanisms, has been performed, and in particular, no unique diagnosis marker of chronic graft rejection has been disclosed.

In PCT applications WO2005/070086 (2) and WO2006/131537 (3), the inventors compared, using microarray technology, the expression profiles of tolerant grafted subjects and non-tolerant grafted subject. Several signatures permitting to discriminate between tolerant and non-tolerant (including subjects undergoing chronic rejection) subjects were described. However, this study was mainly directed to the identification of tolerant subjects, in which the immunosuppressive treatment might be stopped or decreased, rather than of subjects undergoing chronic rejection. In addition, although several genes were found upregulated in subjects undergoing chronic rejection, no unique diagnosis marker of chronic graft rejection, which would alone allow the discrimination between subjects with stable graft function and subjects undergoing chronic rejection, has been disclosed.

Scherer et al (4) describe the comparison, using microarray technology, of the expression profiles of kidney biopsies of subjects who, six months after the biopsy, either still had normal kidney function or were undergoing chronic rejection. They identified a signature of ten genes permitting to discriminate between the two groups of subjects. However, no confirmation of the diagnosis power of this signature in blood is described. In addition, no unique diagnosis marker of chronic graft rejection, which would alone allow the discrimination between subjects with stable graft function and subjects undergoing chronic rejection, has been disclosed.

There is thus still a need for a simple, accurate, reliable and non invasive method for diagnosing chronic graft rejection in a transplanted subject, based on the analysis of a biological sample, preferably a blood sample.

The proteasome is a multicatalytic proteinase complex with a highly ordered ring-shaped 20S core structure. The core structure is composed of 4 rings of 28 non-identical subunits; 2 rings are composed of 7 alpha subunits and 2 rings are composed of 7 beta subunits. Proteasomes are distributed throughout eukaryotic cells at a high concentration and cleave peptides in an ATP/ubiquitin-dependent process in a non-lysosomal pathway. An essential function of a modified proteasome, the immunoproteasome, is the processing of class I MHC peptides. The immunoproteasome is also involved in many cellular functions such as cell cycle regulation, cell proliferation and apoptosis. PSMB 10 (proteasome beta 10 subunit, see Entrez Gene GeneID: 5699) is an interferon inducible catalytic subunit of the immunoproteasome.

The inventors analyzed by DNA microarray technology the blood and graft transcription profiles of kidney grafted patients with chronic rejection or with stable graft function. They further studied the blood and graft transcription profiles of kidney or heart grafted rats with chronic rejection, acute rejection, or rats grafted with a syngenic graft and normal ungrafted rats. They then identified PSMB10 as being significantly upregulated in situations of chronic rejection, both in rat models and in human patients. The upregulation of PSMB10 was confirmed both in blood and in biopsies. In addition, the inventors also confirmed that the mere measure of PSMB10 expression level in blood or in biopsy is sufficient to discriminate between subjects with stable graft function and subjects undergoing chronic rejection.

In addition to the problem of diagnosing chronic graft rejection, it would also be very useful to find treatments of chronic rejection. Indeed, while acute rejection is usually well treated using currently available commercial drugs, there is currently no efficient treatment of chronic rejection. Most of the time, when the process of chronic rejection is started, it cannot be stopped and results in graft loss.

Acute rejection is an early process, which may appear in a few months following transplantation. It can have humoral and/or cellular mechanisms involving mononuclear, cytotoxic and Th cells, and cytokines.

In contrast, chronic rejection appears later and is due to several factors (proteinuria, hypertension, slow and progressive degradation of graft function) and chronic histological lesions mediated by antibodies. Although the mechanisms of chronic rejection are still not well understood, it is clear that they are distinct from the mechanisms of acute rejection. In particular, there is evidence that both immune and nonimmune events are responsible. In addition, none of currently available drugs, which have an effect on acute rejection, has been found to be efficient in chronic rejection.

There is thus a need for a new treatment which would be efficient for treating chronic graft rejection.

Proteasome inhibitor dipeptide boronic acid (DPBA) has been studied in an acute graft rejection model in mice and proposed as a new inhibitor of graft rejection by Luo et al (5). In this article, C57BL/6 mice (H-2^{b}) were used as recipients and 2- to 4-month-old BALB/c mice (H-2^{d}) were used as heart donors. Since the H-2 restriction of C57BL/6 and BALB/c mice is different, in the absence of immunosuppressive treatment, such an allograft with a strong MHC incompatibility induces acute rejection and not chronic rejection.

The authors have then tested the ability of DPBA to prevent or delay acute graft rejection in this model. DPBA treated mice had an increased graft survival compared to non treated control mice. The author concluded that a proteasome inhibitor can be used as an effective immunosuppressant in organ transplantation.

In addition, DPBA also permitted to suppress ongoing acute graft rejection and the authors concluded that it might be very useful in clinical transplantation in treating rejection episodes.

Although the word "acute" is not used in this study, it is clear from the mouse model used, in which an allogenic organ is transplanted, that only acute rejection has been studied in this article.

As mentioned before, drugs efficient for preventing or treating acute rejection have at the present time not been found as useful for treating chronic rejection.

In addition, several patent applications suggested that proteasome inhibitors might be used for treating or preventing graft rejection (see for instance WO 99/22729, WO 2004/014882 and WO 2006/099261, ref 6-8). However, none of these applications described that these compounds could be used for treating the very particular and distinct process of chronic graft rejection. Indeed, as mention above, contrary to acute rejection, chronic rejection is based on both immune-related and non immune-related processes, and the mere ability of proteasome inhibitors to inhibit activated lymphocytes proliferation would not be sufficient to prevent or treat chronic rejection. Moreover, none of these documents effectively describes the ability of proteasome inhibitors to treat or prevent even acute graft rejection, such an ability of proteasome inhibitors being merely speculative.

However, by studying a rat chronic rejection model, the inventors surprisingly found that proteasome inhibitors are efficient for treating, not only acute rejection as described in Luo et al (5), but also for treating chronic graft rejection.

### DESCRIPTION OF THE INVENTION

The invention thus relates to a method for diagnosing chronic rejection of a grafted organ in a subject from a biological sample of said subject, comprising:
(a) determining in vitro an expression level value for PSMB10 in said subject biological sample,
(b) comparing said value to:
   - at least one reference expression level value for PSMB 10 in at least one reference sample, or
   - a reference threshold value, and
(c) diagnosing if said subject is or not undergoing chronic rejection of said grafted organ.

Graft rejection has been categorized into three subsets (hyperacute, acute or chronic rejection) depending on the onset of graft destruction. This is clinically important due to the fact that all three subsets have different pathways by which implants undergo tissue death. "Hyperacute rejection" is the term applied to very early graft destruction, usually within the first 48 hours. It occurs when preformed antibodies are present in the recipient's serum that are specific for donor antigens expressed on graft vascular endothelial cells. Thus, tissue death is due to a humoral immune response. "Acute rejection" has an onset of five days to three months after transplantation and can have humoral and/or cellular mechanisms involving mononuclear, cytotoxic and Th cells, and cytokines. "Chronic rejection" occurs months to years after transplantation. Slowly deteriorating graft function caused by fibrosis of the graft parenchyma and widespread arteriopathy are the hallmarks of chronic rejection that lead to loss of function and eventual graft loss. The mechanisms of chronic rejection are still poorly understood. However, there is evidence that both immune and nonimmune events are responsible.

The method according to the invention is performed on a biological sample from a grafted subject. Said subject may be grafted with any organ. In an advantageous embodiment, the grafted organ is kidney, liver, pancreas or heart, more preferably kidney. Said subject may be a human being or another mammal.

The method according to the invention is performed on a grafted subject's biological sample. A "biological sample" may be any sample that may be taken from a grafted subject, such as a serum sample, a plasma sample, a urine sample, a blood sample, a lymph sample, or a biopsy. Such a sample must allow for the determination of the expression level value of PSMB10. Preferred biological samples for the determination of an expression profile include samples such as a blood sample, a lymph sample, or a biopsy. Preferably, the subject biological sample is a blood sample, more preferably a peripheral blood sample comprising peripheral blood mononuclear cells (PBMC). Indeed, such a blood sample may be obtained by a completely harmless blood collection from the grafted patient and thus allows for a non-invasive diagnosis of a graft tolerant or non-tolerant phenotype. In particular, this permits to plan regular controls of the patient, since quality of life is preserved and there is no risk for the grafted organ.

The expression level value for PSMB10 may be determined using any suitable technology known in the art. In particular, the expression level value for PSMB10 may be determined at the nucleic level, by measuring the amount of nucleic acid transcripts of PSMB10, or at the proteic level, by measuring the amount of PSMB10 protein.

In a preferred embodiment, the expression level value for PSMB10 is determined at the nucleic level, by measuring the amount of nucleic acid transcripts of PSMB10.

The amount of nucleic acid transcripts can be measured by any technology known by a man skilled in the art. In particular, the measure may be carried out directly on an extracted messenger RNA (mRNA) sample, or on retrotranscribed complementary DNA (cDNA) prepared from extracted mRNA by technologies well-know in the art. From the mRNA or cDNA sample, the amount of nucleic acid transcripts may be measured using any technology known by a man skilled in the art, including nucleic microarrays, quantitative PCR, and hybridization with a labelled probe. The PSMB10 gene and mRNA sequences of humans or other species are available from public sequence databases such as the NCBI database. In particular, all information on human PSMB10 gene is available on PubMed EntrezGene database under number GeneID 5699. PSMB10 genomic sequence is available under Genbank accession numbers NC_000016.8 and NM_002801.2 respectively. Further PSMB10 sequences of other species may be obtained from Genbank, such as murine or rat sequences for instance (see PubMed EntrezGene GeneID 19171 and 291983 respectively). Based on these sequences, it is easy for any skilled artisan to design suitable reagents for measuring PSMB 10 mRNA expression levels in any mRNA or cDNA sample.

In a preferred embodiment, the expression profile is determined using quantitative PCR. Quantitative, or real-time, PCR is a well known and easily available technology for those skilled in the art and does not need a precise description.

In a particular embodiment, which should not be considered as limiting the scope of the invention, the determination of the expression profile using quantitative PCR may be performed as follows. Briefly, the real-time PCR reactions are carried out using the TaqMan Universal PCR Master Mix (Applied Biosystems). 6 µl cDNA is added to a 9 µl PCR mixture containing 7.5 µl TaqMan Universal PCR Master Mix, 0.75 µl of a 20X mixture of probe and primers and 0.75µl water. The reaction consisted of one initiating step of 2 min at 50 deg. C, followed by 10 min at 95 deg. C, and 40 cycles of amplification including 15 sec at 95 deg. C and 1 min at 60 deg. C. The reaction and data acquisition can be performed using the ABI PRISM 7900 Sequence Detection System (Applied Biosystems). The number of template transcript molecules in a sample is determined by recording the amplification cycle in the exponential phase (cycle threshold or C_{T}), at which time the fluorescence signal can be detected above background fluorescence. Thus, the starting number of template transcript molecules is inversely related to C_{T}.

In one embodiment, the diagnosis of chronic graft rejection is carried out thanks to the comparison of the obtained expression level value of PSMB10 in the tested sample with a reference expression level value of PSMB10 in a reference sample in step (b).

A "reference sample" is a biological sample from a subject with a known particular graft state. In a preferred embodiment, said reference sample to which the expression level value of PSMB10 in the tested sample is compared is from a grafted subject with stable graft function. Several reference samples from several grafted subjects with stable graft function may be used instead of only one. Preferably, the obtained expression level value for PSMB10 in said subject biological sample is compared to a reference expression level value for PSMB10 in a corresponding biological sample from a grafted subject with stable graft function. By a "corresponding biological sample" is meant a biological sample of corresponding physiological nature. Indeed, a PSMB10 expression level value obtained in a test blood sample will be preferably compared to reference PSMB10 expression level value(s) obtained from reference blood samples. Accordingly, a PSMB 10 expression level value obtained in a test biopsy will be preferably compared to reference PSMB10 expression level value(s) obtained from reference biopsies.

In a preferred embodiment, both a sample from a grafted subject with stable graft function and from a grafted subject who is undergoing chronic rejection are used as reference samples. Here also, several reference samples of each type (grafted subject with stable graft function versus grafted subject undergoing chronic rejection) may be used instead of only one of each. Preferably, the obtained expression level value for PSMB10 in said subject biological sample is compared both to a reference expression level value for PSMB10 in a corresponding biological sample from a grafted subject with stable graft function and to a reference expression level value for PSMB10 in a corresponding biological sample from a grafted subject who is suffering from chronic rejection.

The comparison of a tested subject expression profile with said reference expression profiles can be done using PAM (predictive analysis of microarrays) statistical method. A non supervised PAM 3 classes statistical analysis is thus performed. Briefly, reference expression level values of PSMB10 is reference samples (from grafted subject(s) with stable graft function and/or from grafted subject(s) undergoing chronic rejection) and the expression level value of PSMB10 in the tested subject are subjected to a clustering analysis using non supervised PAM 3 classes statistical analysis. Based on this clustering, a cross validation (CV) probability may be calculated (CV_{CR}), which represents the probability that the tested subject is undergoing chronic rejection. The diagnosis is then performed based on the CV_{CR} probabilities. Preferably, a subject is diagnosed as undergoing chronic graft rejection if the CV_{CR1} probability is of at least 0.5, at least 0.6, at least 0.7, at least 0.75, at least 0.80, at least 0.85, more preferably at least 0.90, at least 0.95, at least 0.97, at least 0.98, at least 0.99, or even 1.00.

In another embodiment, since only the expression level of PSMB 10 is determined, the PSMB 10 expression level value of the test sample may be compared in step (b) to a reference threshold value in order to perform the diagnosis. Such a threshold value may vary depending on the grafted organ, the type of tested biological sample, or on the method used for determining PSMB 10 expression level. However, for particular experimental conditions (same grafted organ, same type of test biological sample, same method for determining PSMB 10 expression level in the tested biological sample), said threshold value may be determined based on a reference pool of patients comprising both a population of patients with stable graft function and a population of patients undergoing chronic rejection). By measuring the PSMB10 expression level values of these patients, a reference threshold value T can be determined with the following features:
- all or most reference patients with stable graft function have PSMB 10 expression level values inferior to T, and
- all or most reference patients undergoing chronic rejection have PSMB 10 expression level values superior to T.

Additional details concerning the method for determining such a reference threshold value are indicated in Example 2.

In particular, if the grafted organ is kidney, and PSMB 10 expression values are determined at the nucleic level using quantitative PCR as described above, then the reference threshold value permitting to discriminate patients with stable graft function from patients undergoing chronic rejection is 1.57 if the tested biological sample is blood, and 1.44 if the tested biological sample is a kidney biopsy. This means that all further tested subjects with a PSMB10 expression level value superior to 1.57 in blood or 1.44 in a kidney biopsy should be considered as undergoing chronic rejection.

However, the higher is PSMB 10 expression level value, the higher is the probability that the tested subject is undergoing chronic rejection. As a result, if the tested sample is a blood sample, and if the PSMB10 expression level value of the test sample is of at least 1.57, preferably at least 1.58, at least 1.59, at least 1.60, more preferably at least 1.65, at least 1.70, still more preferably at least 1.75, at least 1.80, at least 1.85, at least 1.90, at least 1.95, or at least 2.00, then the tested subject is diagnosed as undergoing chronic rejection. Otherwise, said tested subject is diagnosed as having stable graft function. Alternatively, if the tested sample is a biopsy sample, and if the PSMB10 expression level value of the test sample is of at least 1.44, preferably at least 1.46, at least 1.48, at least 1.50, more preferably at least 1.55, at least 1.60, at least 1.65, at least 1.70, still more preferably at least 1.75, at least 1.80, at least 1.85, at least 1.90, at least 1.95, or at least 2.00, then the tested subject is diagnosed as undergoing chronic rejection. Otherwise, said tested subject is diagnosed as having stable graft function.

In an embodiment of a method according to the invention, said method may further comprise determining in said subject biological sample the expression level of at least one additional gene. In particular, the expression level of further genes that are known to be associated with graft tolerance or graft rejection may be measured in addition to PSMB10. Examples of such genes include genes identified in applications WO 2005/070086, W02006/131537, and WO 2006/099421 (9-11)

In a particular embodiment of any method according to the invention described above, said method may further comprise determining from a biological sample of the subject at least one additional parameter useful for the diagnosis. Such "parameters useful for the diagnosis" are parameters that cannot be used alone for a diagnosis but that have been described as displaying significantly different values between grafted subjects with stable graft function and subjects in chronic rejection and may thus also be used to refine and/or confirm the diagnosis according to the above described method according to the invention. They may notably be selected from:
- standard biological parameters specific for said subject grafted organ type,
- phenotypic analyses of peripheral blood mononuclear cells (PBMC), and
- qualitative and/or quantitative analysis of PBMC immune repertoire.

According to the invention, "standard biological parameters specific for said subject grafted organ type" means biological parameters that are usually used by clinicians to monitor the stability of the status of grafted subjects and to detect graft rejection. These standard biological parameters specific for said subject grafted organ type usually comprise serum or plasma concentrations of particular proteins, which vary depending on the grafted organ type. However, these standard biological parameters specific for said subject grafted organ type are, for each organ type, well known of those skilled in the art.

For instance, standard biological parameters specific for kidney include serum or plasma urea and creatinine concentrations. In a healthy subject, the serum creatinine concentration is usually comprised between 40 to 80 µmol/L for a woman and 60 to 100 µmol/L for a man, and the serum urea concentration between 4 to 7 mmol/L. A serum creatinine concentration superior to 80 µmol/L for a woman and to 100 µmol/L for a man, or a serum urea concentration superior to 7 mmol/L may confirm a chronic rejection status.

For instance, for liver transplantation, standard biological parameters include serum or plasma concentrations of gamma glutamyl transpeptidase (GGT), aspartate aminotransferase (AST), alanine aminotransferase (ALT), lactate dehydrogenase (LDH), and bilirubin (total or conjugated).

These standard biological parameters have the advantage of being easily measurable from a blood sample, but are not sufficient to establish a precise graft tolerant or non-tolerant diagnosis, and are also not enough sensitive to allow an early chronic rejection diagnosis. However, they may confirm the diagnosis obtained using PSMB 10 expression levels.

The phenotypic analyses of peripheral blood mononuclear cells (PBMC) may comprise various types of phenotypic analysis. In particular they may comprise:
- measuring the percentage of CD4⁺ CD25⁺ T cells in peripheral blood lymphocytes, which may be performed by any technology known in the art, in particular by flow cytometry using labelled antibodies specific for the CD4 and CD25 molecules. Preferably, the percentage of CD4⁺ CD25⁺ T cells in peripheral blood lymphocytes of a subject undergoing chronic rejection is significantly lower (p < 0.05) from that of a healthy volunteer (12).
- determining the cytokine expression profile of T cells, which may be performed using any technology known in the art, including quantitative PCR and flow cytometry analysis. Preferably, the oligoclonal Vβ families of a grafted subject undergoing chronic rejection express increased levels compared to a healthy volunteer of TH1 or TH2 effector molecules, including interleukin 2 (IL-2), interleukin 8 (IL-8), interleukin 10 (IL-10), interleukin 13 (IL-13), transforming growth factor beta (TGF-β), interferon gamma (IFN-γ) and perforin, whereas oligoclonal Vβ families of a grafted subject with stable graft function do not express increased levels of those effector molecules compared to a healthy volunteer (13).

The analysis of PBMC immune repertoire consists advantageously in the qualitative and quantitative analysis of the T cell repertoire (13), such as the T cell repertoire oligoclonality and the level of TCR transcripts or genes.

The T cell repertoire oligoclonality may be determined by any technology enabling to quantify the alteration of a subject T cell repertoire diversity compared to a control repertoire. Usually, said alteration of a subject T cell repertoire diversity compared to a control repertoire is determined by quantifying the alteration of T cell receptors (TCR) complementary determining region 3 (CDR3) size distributions. In a healthy subject, who can be considered as a control repertoire, such a TCR CDR3 size distribution displays a Gaussian form, which may be altered in the presence of clonal expansions due to immune response, or when the T cell repertoire diversity is limited and reaches oligoclonality.

The level of TCR expression at the genomic, transcriptionnal or protein level is preferably determined independently for each Vβ family by any technology known in the art. For instance, the level of TCR transcripts of a particular Vβ family may be determined by calculating the ratio between these Vβ transcripts and the transcripts of a control housekeeping gene, such as the HPRT gene. Preferably, in a graft tolerant subject, a significant percentage of Vβ families display an increase in their transcript numbers compared to a normal healthy subject.

An example of methods to analyze T cell repertoire oligoclonality and/or the level of TCR transcripts, as well as scientific background relative to T cell repertoire, are clearly and extensively described in WO 02/084567 (14), which is herein incorporated by reference. Preferably, a graft tolerant subject, as well as a subject in chronic or acute rejection, displays a T cell repertoire with a significantly higher oligoclonality than a normal healthy subject.

Such additional parameters may be used to confirm the diagnosis obtained using PSMB10 expression level. For instance, when the subject is a kidney grafted subject, certain values of the standard biological parameters may confirm a chronic rejection diagnosis: if the serum concentration of urea is superior to 7 mmol/L or the serum concentration of creatinine is superior to 80 µmol/L for a female subject or 100 µmol/L for a male subject, then the tested subject is diagnosed as undergoing chronic rejection.

The invention also concerns a kit for the diagnosis of a chronic graft rejection, comprising at least one reagent for the determination of PSMB10 expression level in a biological sample, wherein the reagents present in said kit permit to determine the expression level of at most 500 distinct genes.

By "a reagent for the determination of PSMB10 expression level" is meant a reagent which is specifically intended for the specific determination of the PSMB10 expression level. This definition excludes generic reagents useful for the determination of the expression level of any gene, such as taq polymerase or an amplification buffer, although such reagents may also be included in a kit according to the invention.

Such a kit for the diagnosis of a chronic graft rejection may further comprise instructions for carrying out the diagnosis.

Such a kit for the diagnosis of a chronic graft rejection may also further comprise at least one reagent for the determination of at least one additional parameter useful for the diagnosis such as the expression level of other genes associated to graft tolerance or rejection, standard biological parameters specific for said subject grafted organ type, phenotypic analyses of PBMC (notably the percentage of CD4⁺ CD25⁺ T cells in peripheral blood lymphocytes and the cytokine expression profile of T cells), and quantitative and/or qualitative analysis of PBMC immune repertoire (such as the T cell repertoire oligoclonality and the level of TCR transcripts).

In any kit for the diagnosis of a chronic graft rejection according to the invention, the reagent(s) for the determination of PSMB10 expression level preferably include specific amplification primers and/or probes for the specific quantitative amplification of transcripts of PSMB10, and/or a nucleic microarray for the detection of PSMB10. The determination of the expression profile may thus be performed using quantitative PCR and/or a nucleic microarray, preferably an oligonucleotide microarray.

In any case, the reagents present in such a kit for the diagnosis of a chronic graft rejection according to the invention permit to determine the expression level of at most 500, preferably at most 300, more preferably at most 200, more preferably at most 150, even more preferably at most 100, even more preferably at most 75 distinct genes, thus excluding for instance pangenomic microarrays.

In addition, the instructions for carrying out the diagnosis preferably include:
- at least one reference expression level value for PSMB 10 in at least one reference sample. In a preferred embodiment, said reference expression level value for PSMB10 is the expression level value for PSMB10 in a corresponding biological sample from a grafted subject with stable graft function. In another preferred embodiment, the instructions include both a reference expression level value for PSMB10 in a corresponding biological sample from a grafted subject with stable graft function and a reference expression level value for PSMB10 in a corresponding biological sample from a grafted subject who is suffering from chronic rejection; or
- at least one reference threshold value as defined above.

The invention also concerns a nucleic acid microarray comprising at least one nucleic acid specific for the PSMB10 gene, wherein said nucleic acid microarray comprises nucleic acids specific for at most 500 distinct genes.

Said nucleic acid microarray may further comprise at least one nucleic acid specific for at least one gene associated with graft tolerance or rejection as described above. However, in any case, said nucleic acid microarray comprises nucleic acids specific for at most 500, preferably at most 300, more preferably at most 200, more preferably at most 150, even more preferably at most 100, even more preferably at most 75 distinct genes.

In a preferred embodiment, said nucleic acid microarray is an oligonucleotide microarray.

The invention further relates to a proteasome inhibitor, for use as a medicament for treating chronic graft rejection.

The invention also concerns the use of a proteasome inhibitor for preparing a medicament for treating chronic graft rejection.

The invention also relates to a method for treating chronic graft rejection in a patient in need therof, comprising administering to said patient an effective amount of a proteasome inhibitor.

Several families of proteasome inhibitors have been described. They can be broadly categorized into two groups: synthetic analogs and natural products. The proteasome has several proteolytic activities. The three main activities are referred to as the chymotrypsin-like (CT-L), trypsin-like (T-L), and post-glutamyl peptide hydrolyzing (PGPH) activities, respectively. In addition to these well-characterized proteolytic activities, the 20S proteasome is also known to possess two other proteolytic activities, i.e., branched-chain amino acid-preferring (BrAAP) and small neutral amino acid-preferring (SNAAP) activities.

Families of proteasome inhibitors and their particular features, notably their specificity for one or more proteolytic activities, are described in Myung et al (15) and in Guédat et al (16), which more particularly describes patented small molecule inhibitors in the ubuquitin proteasome system, and notably patented small molecule proteasome inhibitors. A summary of compounds described in these documents is provided below. In addition, all proteasome inhibitors described in these documents are herein incorporated by reference.

Synthetic inhibitors are peptide-based compounds with diverse pharmacophores. These include peptide benzamides, peptide α-ketoamides, peptide aldehydes, peptide α-ketoaldehydes, peptide vinyl sulfones, and peptide boronic acids.

Known synthetic peptide aldehydes with proteasome inhibitory capacities include compounds **1** to **7** below:

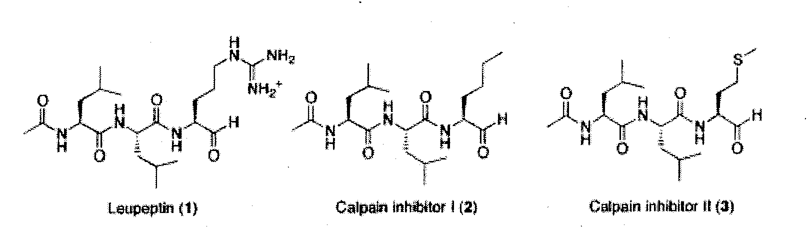

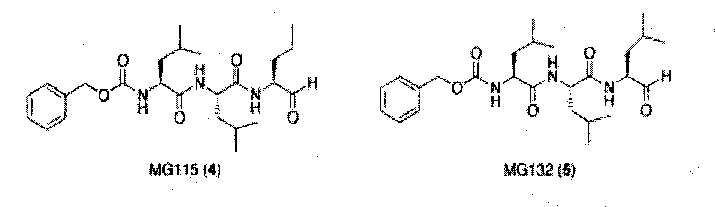

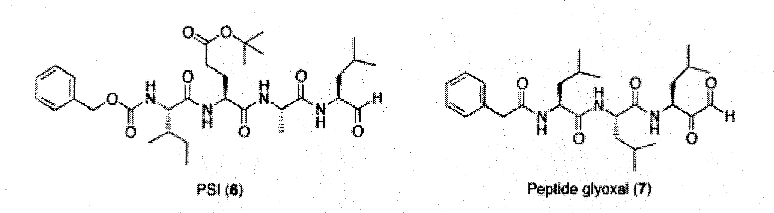

Non-reactive peptide inhibitors, such as α-ketocarbonyl and boronic ester derivatives, indanone dipeptide benzamides, and P'-extended α-ketoamides have also been developed (see for instance compounds 8 to **11** below):

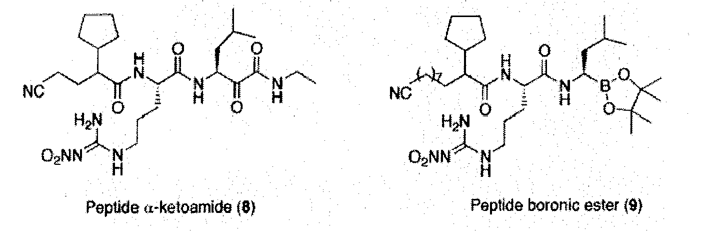

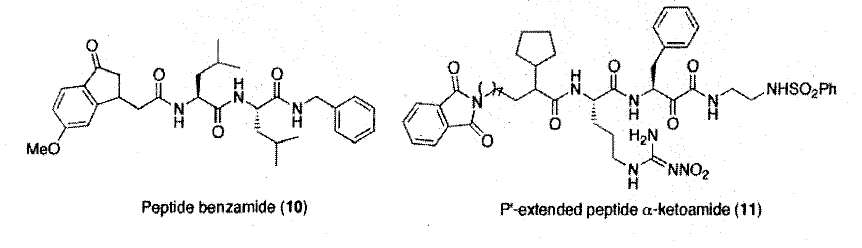

Non-aldehyde irreversible proteasome inhibitors have also been developed, such as tripeptides α',β'-epoxyketones and vinyl sulfones (see for instance compounds **12** and **13** below).

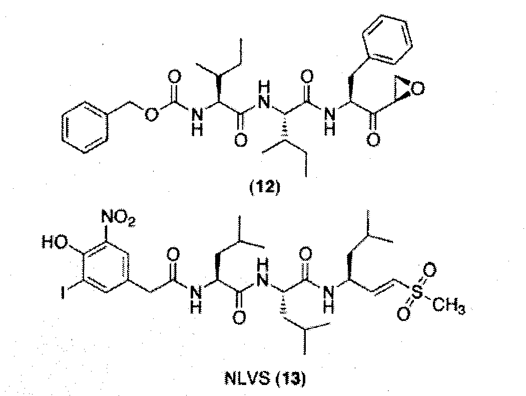

Another class of peptide-based inhibitors exploits the boronic acid functional group. The most well characterized compound of this class is a dipeptide boronic acid PS-341, also known as bortezomib and commercialized under the name Velcade® (see compound **14** below):

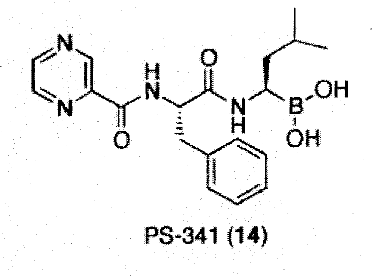

Close related analogs of bortezomib, such as boronic acid derivatives, benzylmalonic- and amino acid-based derivatives, and boronic ester have also been found to have proteasome inhibitory capacities, such as thise described in WO2003/033506, WO2003/033507, WO2006/086600, WO2005/21558, and WO2005/016859 (17-21). Other compounds with potential proteasome inhibitory capacities, with boronic acid or ester function, such as lactam derivatives, have also been described in WO2004/064755 (22).

While the synthetic inhibitors mentioned above have been rationally designed, synthesized, and optimized, nature has also provided selective and potent proteasome inhibitors. Natural product proteasome inhibitors display a variety of scaffolds of core structures and pharmacophores. Known natural product proteasome inhibitors include linear peptide epoxyketones, peptide macrocycles, γ-lactam thiol ester, and epipolythiodioxopiperazine toxin.

For example, lactacystin (see compound **15** below) is a Streptomyces lactacystinaeus metabolite that was discovered on the basis of its ability to induce neurite outgrowth in the murine neuroblastoma cell line Neuro-2a. The active component of lactacystin is the clasto-lactacystin β-lactone (see compound **16**) that is a rearrangement product of lactacystin in aqueous condition.

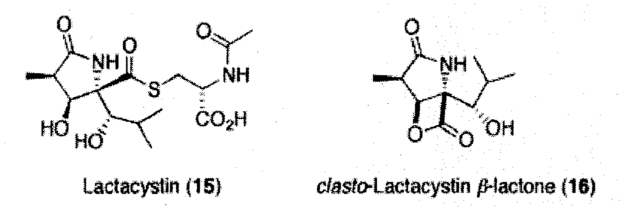

Epoxomicin (see compound **17** below) from an unidentified actinomycete strain No. Q996-17. Epoxomicin (compound **17**) is a member of growing family of linear peptide α',β'-epoxyketone natural products (see for instance compounds **17** to **23** below).

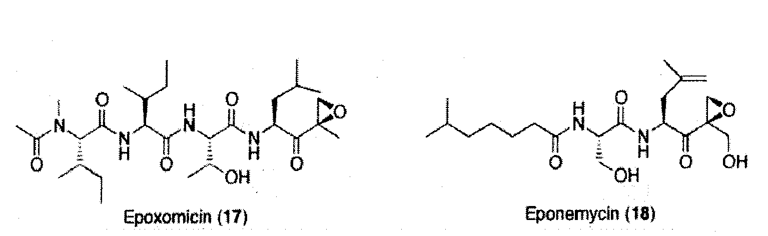

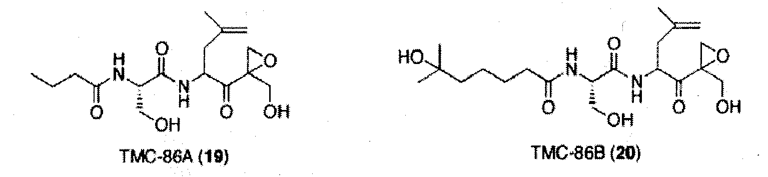

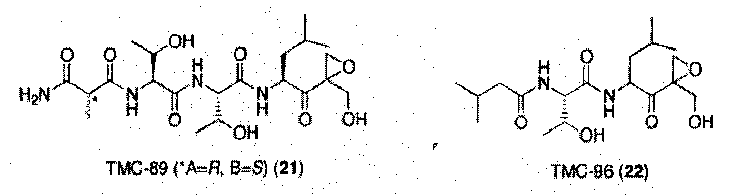

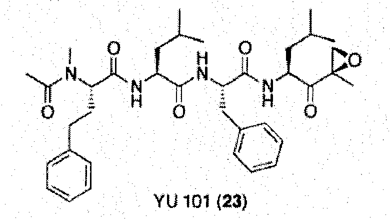

Another epoxomicin derivative is carfilzomib (also known as PR-171, see compound **27** below), described in WO2006/017842 (23):

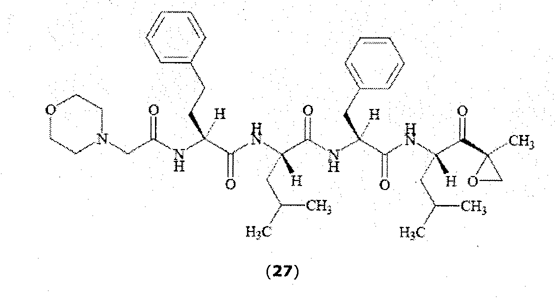

Other analogs of eponemycin and epoxomicin have been also reported to inhibit the proteasome in WO2003/059898 (24).

While lactacystin and epoxomicin are the best known natural product inhibitors of the 20S proteasome, there have been other potent natural product proteasome inhibitors with novel structures. These include a series of TMC-95 (see compounds **24** below) from the fermentation broth of Apiospora montagnei Sacc. TC 1093. Despite their unusual macrocyclic peptide architecture, these compounds possess potent inhibitory activity toward the CT-L activity of the 20S proteasome.

Another unexpected class of natural product proteasome inhibitors includes gliotoxin (see compound **25** below), a member of the fungal epipolythiodioxopiperazine toxins that are characterized by a heterobicyclic core containing a disulfide bridge(s), which is responsible for its inhibitory action.

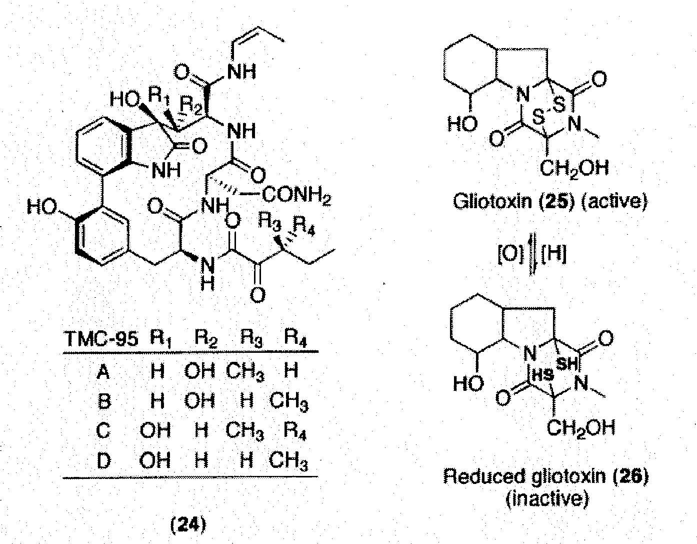

Another class of natural product proteasome inhibitors includes salinosporamide A (NPI-0052) (see compound **28** below), a secondary metabolite derived from a novel obligate marine actinomycete (Salinispora tropicana), and its analogs, as described in WO2006/060809, WO2005/002572 and WO2005/099687 (25-27). NPI-0052 is a highly potent and selective 20S proteasome inhibitor with irreversible activity against all three catalytic sites.

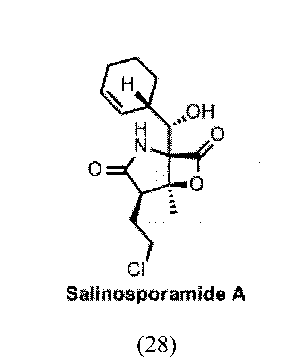

Open analogs of salinosporamide A, such as 2-pyrrolidone derivatives from microorganisms (Streptomyces JS360), have been also described in WO2004/071382 (28).

Other natural compounds, such as (-)-epigallocatechin 3-gallate ((-)-EGCG, see compound **29** below), the most abundant catechin in green tea, act as chemoprotective and anticancer agents by inhibiting the chymotrypsin-like activity of the purified 20S proteasome *in vitro.*

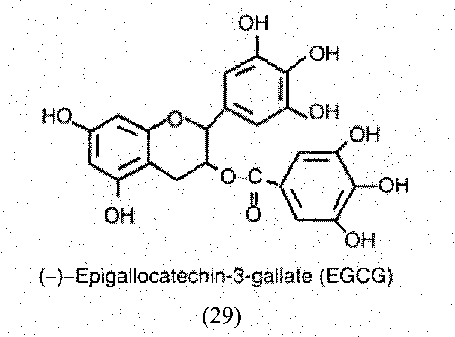

Analogs of (-)-EGCG have also been been described in WO2006/017981 (29).

A great number of proteasome inhibitors are thus already known and may be used in the invention for treating chronic graft rejection. In preferred embodiment of the invention, a proteasome inhibitor for use in treating chronic graft rejection is selected from the group consisting of peptide benzamides, peptide α-ketoamides, peptide aldehydes, peptide α-ketoaldehydes, peptide vinyl sulfones, peptide boronic acids, linear peptide epoxyketones, peptide macrocycles, γ-lactam thiol ester, epipolythiodioxopiperazine toxins, salinosporamide A and analogs thereof, (-)-epigallocatechin 3-gallate and analogs thereof. Advantageously, said proteasome inhibitor for use in treating chronic graft rejection is selected from the group consisting of bortezomib, carfilzomib, and analogs or derivatives thereof as described above.

### DESCRIPTION OF THE FIGURES

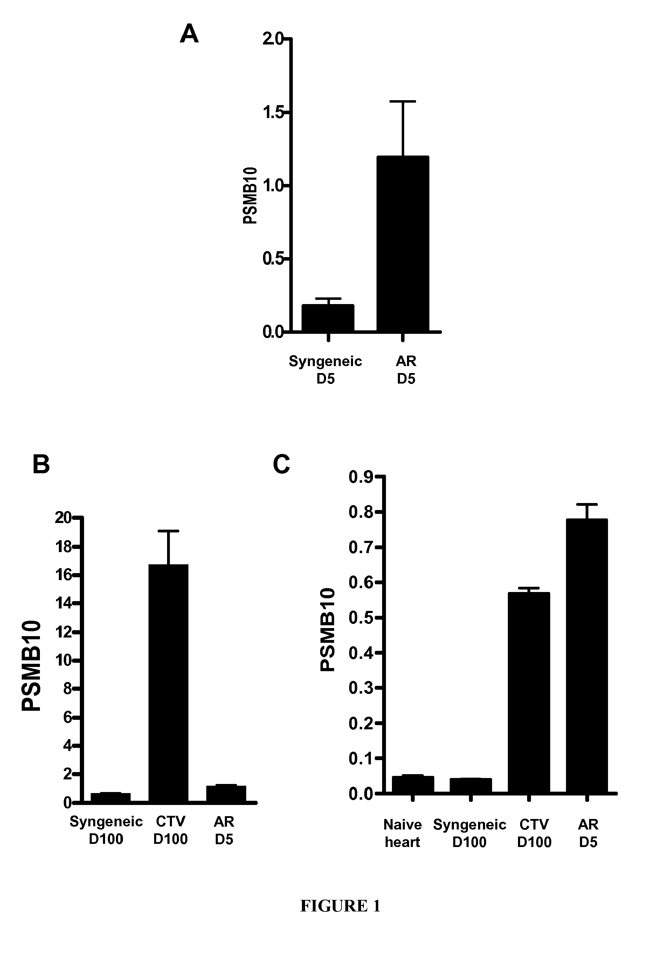

**Figure 1****.** Quantitative PCR measurement of PSMB10 mRNA transcription in (A) syngeneic or acutely rejecting (AR) kidney allografts at Day 5 post transplantation and in (B) the peripheral blood mononuclear cells (PBMC) or (C) cardiac allograft (naive, syngeneic, chronic transplant vasculopathy (CTV) at Day 100 post transplantation or acute rejection (AR) at Day 5 post transplantation) in a rat model of allotransplantation.

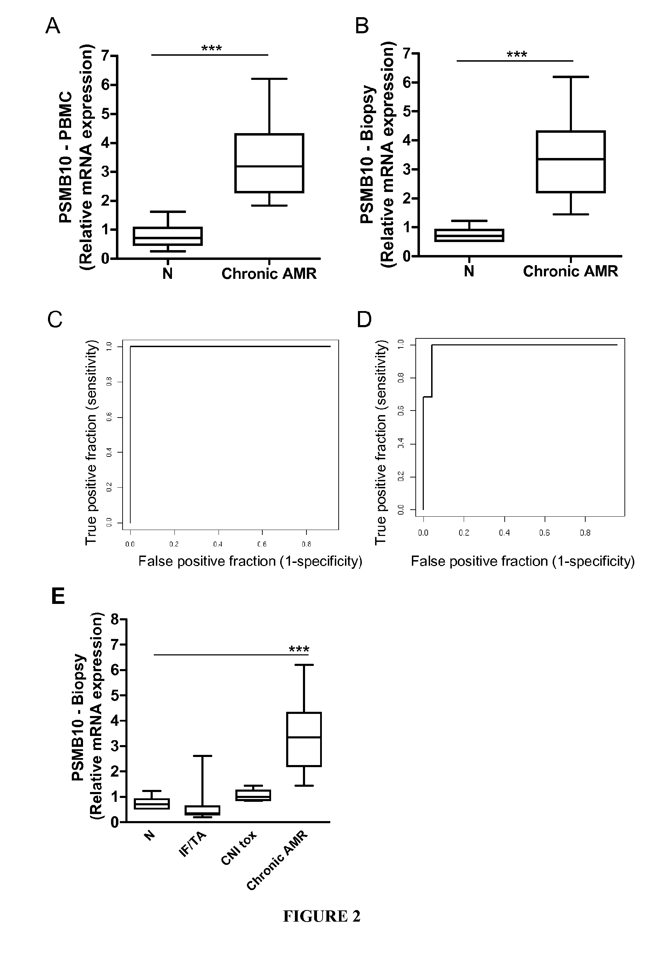

**Figure 2**. PSMB10 expression in **A** peripheral blood mononuclear cells (PBMC) and **B** graft biopsies from renal transplant patients whose graft displays normal histology (N) or signs of chronic antibody-mediated rejection (AMR). Expression levels were measured by quantitative PCR. Receiver-operator-characteristic (ROC) curve analysis of PSMB10 mRNA in **C** peripheral blood mononuclear cells (PBMC) and **D** graft biopsies from the same renal transplant patients included in **A** and **B** (n = 25 for PBMC and n = 43 for biopsies). The ROC is represented as a graphical plot of the sensitivity vs. (1 - specificity) for a binary classifier system as its discrimination threshold is varied. The sensitivity (also referred to as the "true positive fraction") is how good the test is at picking out patients with chronic AMR. Specificity is the ability of the test to pick out patients who do not have chronic AMR. Thus, (1-specificity) is also referred to as the "false positive fraction". The accuracy of the test (i.e. how well the test separates patients with and without chronic AMR) is measured by the area under the ROC curve where an area of 1.0 represents a perfect test. Thus, the ROC curve should climb rapidly towards the upper left hand corner of the graph, meaning that the false negative rate is high and the false positive rate is low. The AUC is 1.0 for PSMB10 in the blood and 0.99 for PSMB10 in the biopsies. **E.** Quantitative PCR measurement of PSMB10 mRNA transcription in human kidney graft biopsies displaying normal histology (N), interstitial fibrosis and tubular atrophy (IFTA), calcineurin inhibitor cytotoxicity (CNI tox) or chronic antibody-mediated rejection.

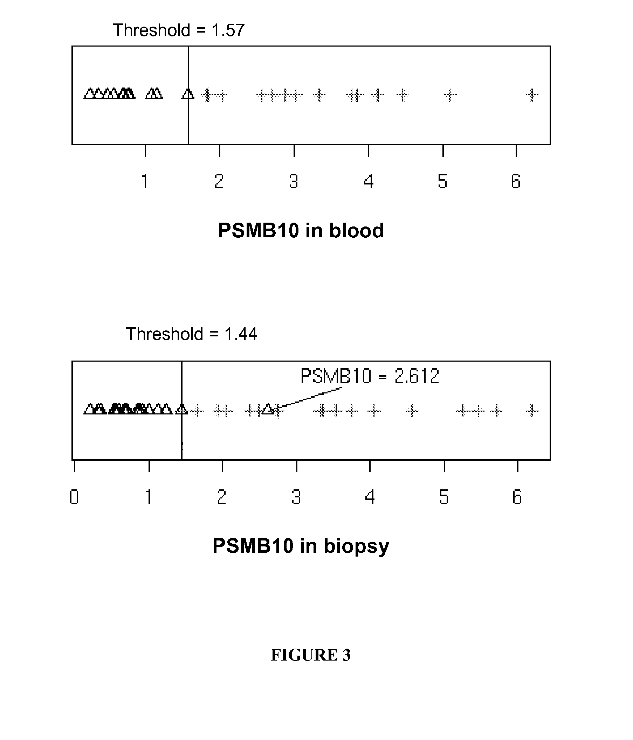

**Figure 3****.** Simple graphical representation of the discriminative power of PSMB10 in the blood (top panel) and biopsies (bottom panel). Values of PSMB10 for patients with chronic rejection are represented as crosses (+) whereas those for stable graft function are represented by triangles (Δ). The threshold values permitting to discriminate patients with stable graft function and patients undergoing chronic graft rejection for both sample types are indicated on the graph.

### EXAMPLES

### EXAMPLE 1. Analysis of rat kidney and heart graft models

### 1.1. Materials and Methods

### Rodent transplant models

### Surgical procedures, treatments and experimental groups

Inbred male adult rats (200-250g) of the LEW.1A (RT1a) and LEW.1W (RT1u) congenic strains were purchased from Janvier (Le Genest-Saint-Isle, France) and maintained in an animal facility under standard conditions according to the European and Institutional Guidelines.

Kidney transplantation: The model used was that of kidney allotransplantation from LEW.1W donors to MHC-mismatched LEW.1A recipients. Kidney transplantations were performed aseptically and a binephrectomy was performed 7 days after transplantation as previously described (3). Rejection, indicated by the death of the binephrectomized rat, was confirmed by histology. Blood urea and creatinine and urine protein: creatinine ratios were measured throughout the post-transplant period. Blood urea <8 mmol/L and blood creatinine <40 mmol/L were considered as normal (2).

Heart transplantation: The model used was that of heart allotransplantation from LEW.1W donors to MHC-mismatched LEW.1A recipients. Syngeneic transplants (LEW.1A donors to LEW.1A recipients) were performed as controls. Heterotopic cardiac allografts were performed as previously described (8).

### Sample preparation

Rat samples: Organs were harvested at day 100 or 1 day before rejection. Blood was collected by cardiac puncture into a heparinized syringe and peripheral blood mononuclear cells were separated on a Ficoll® gradient then stored in TRIzol® reagent (Invitrogen, Cergy Pontoise, France). Sections of the spleen and kidney graft were either snap frozen in liquid nitrogen for future RNA extraction, or embedded in O.C.T. compound (Tissue Tek; Miles Laboratories, Elkhart, IN) and snap-frozen in liquid nitrogen for future immunohistochemistry analysis. Blood cell contamination was avoided by perfusing the organ with PBS. All samples were stored at -70°C until use. Quality and quantity of RNA was determined using an Agilent 2100 BioAnalyzer (Palo Alto, CA). RNA was reverse transcribed into cDNA using an RT-Omniscript kit (QIAGEN) according to the manufacturer's instructions.

### RNA extraction and reverse transcription

Rat samples: Total RNA from rat kidney grafts and PBMC was prepared using the TRIzol® (Invitrogen, Cergy Pontoise, France) extraction method. RNA quantity and quality were determined using a nanodrop spectrophotometer and an Agilent 2100 bioanalyzer. Ten µg of total RNA was set aside for microarray analyses (see below). The remaining RNA was treated with DNase (Roche, Indianapolis, USA) to remove genomic DNA and reverse transcribed into first strand cDNA using polydT oligonucleotide and Moloney murine leukemia virus reverse transcriptase (Invitrogen). Quality of the mRNA was systematically controlled on a 1% agarosis gel.

### Real-time quantitative PCR

Real-time quantitative PCR (qPCR) was performed in an Applied Biosystems GenAmp 7700 sequence detection system (Applied Biosystems, Foster City, CA) using a commercially available primer and probe set for human and rat PSMB10 (Applied Biosystems). Hypoxanthine phosphoribosyl transferase (HPRT: human: Hs99999909_m1; rat: Rn01527838_g1) was used as an endogenous control to normalize RNA starting quantity. Relative expression between a given sample and a reference sample was calculated according to the 2 method (ABI PRISM 7900 user bulletin, PE Applied Biosystems, Foster City 2:11-24, 1997).

### Microarray experiments

*RNA preparation, amplification and hybridization:* 10 µg of total RNA were cleaned up using Rneasy columns (QIAGEN). RNA quantity and quality were determined using a nanodrop spectrophotometer and an Agilent 2100 bioanalyzer. A rRNA 28S/18S ratio of 1.0 ±0.1 was considered as acceptable for RNA amplification. The Applied Biosystems (Applied Biosystems, Foster City, CA, USA) rat Genome Survey Microarray (P/N 4337467) used contained 26,857 60-mer oligonucleotide probes representing 27,088 individual rat genes. An additional 3400 control spots were present on the chip to cover various steps in the hybridization process. Digoxigenin-UTP labeled cRNA was generated and amplified from 0.5 µg of total RNA from each sample using an Applied Biosystems Chemiluminescent NanoAmp RT-IVT Labeling Kit (P/N4365715). Array hybridization was performed for 16 hours at 55°C. Chemiluminescence detection, image acquisition and analysis were performed using the Applied Biosystems Chemiluminescence Detection Kit (P/N 436875D), Analyzer (P/N 4338036) and v-1.1 analyzer software (P/N 4336391) according to the manufacturer's protocol.

### Microarray analysis

Microarray raw data were analyzed by R - language and environment for statistical computing and graphics. Genes were identified using the Panther™ Protein Classification System Probe ID database. Raw data were analyzed according p value and Fold Change (FC). FC was calculated for genes that fulfilled the criterion of significance at p<0.05 and the genes were then processed using software available on www.pantherdb.org.

### 1.2. Results

Further to microarray results indicating that PSMB10 might be overexpressed in samples from kidney or heart grafted rats undergoing acute or chronic rejection, quantitative PCR experiments were performed on mRNA from :
- syngeneic or acutely rejecting (AR) kidney allografts at Day 5 post transplantation, and
- peripheral blood mononuclear cells (PBMC) or cardiac allograft (naive, syngneic, chronic transplant vasculopathy (CTV) at Day 100 post transplantation or acute rejection (AR) at Day 5 post transplantation).

In the kidney rat model of allotransplantation, results show that PSMB10 expression levels are upregulated in acutely rejecting kidney allografts at day 5 compared to syngeneic kidney grafts (see Figure 1A). The amplification factor for PSMB10 expression is around 6.

In the heart rat model of allotransplantation, results show that PSMB10 expression levels are upregulated both in acute rejection at day 5 (AR D5) and in chronic transplant vasculopathy at day 100 (CTV D100) compared to syngeneic graft at day 100 in graft samples (see Figure 1C).

In the blood of heart grafted rats, PSMB10 expression level is significantly upregulated in rats with chronic transplant vasculopathy at day 100 (CTV D100) compared to syngeneic grafted rats at day 100 (Figure 1B). The amplification factor is around 34. In contrast, PSMB10 expression level is only slightly upregulated in rats with acute rejection at day 5 (AR D5) compared to syngeneic grafted rats (Figure 1B), with an amplification factor around 2.

### 1.3. Conclusion

The results described above clearly show that PSMB 10 expression levels are significantly upregulated in blood and in grafted organs of rats with chronic transplant vasculopathy in a heart allograft model.

PSMB10 expression levels are also upregulated in grafted hearts or kidneys of rats with acute rejection, but PSMB10 expression level is not significantly upregulated in the blood of rats with acute heart rejection.

In view of the high amplification factors for PSMB10 expression levels in both blood and grafted heart of rats with chronic transplant vasculopathy, the results clearly demonstrate that PSMB10 expression levels, either in blood or in organ biopsy, may be used to discriminate subjects undergoing chronic rejection from subjects with stable graft function.

### EXAMPLE 2. Analysis of kidney grafted human patients

### 2.1. Materials and methods

PSMB10 mRNA was analysed in 42 biopsies classified according to the updated Banff classification criteria as displaying normal histology (N; n = 7), lesions of interstitial fibrosis and tubular atrophy of unknown etiology (IF/TA; n = 9), lesions of calcineurin inhibitor toxicity (CNI tox; n = 7), or Chronic antibody-mediated rejection (AMR; defined by the diagnostic triad of circulating anti-HLA antibody associated with transplant glomerulopathy and deposition of the complement split product C4d in graft biopsies (n = 19).

Human kidney transplant biopsies were taken with a 16 or 18-gauge needle and stored either in RNAlater (QIAGEN, Courtaboeuf, France) or embedded in Tissue Tek (Miles, Elkhart, IN, USA), snap frozen in liquid nitrogen and stored at -80°C. RNA extraction from all biopsies was performed using the QIAgen RNA microextraction kit (QIAGEN) with on-column DNase treatment according to the manufacturers instructions. In the case of Tissue Tek-embedded biopsies, the biopsies were recovered prior to homogenization by a rapid passage in ice-cold RNase-free water. Homogenization was performed with a PT 3100 Polytron® Rotor-Stator (Kinematica, AG, Luzern, Switzerland). RNA quality and quantity was determined using an Agilent 2100 BioAnalyzer (Palo Alto, CA). RNA was reverse transcribed into cDNA using an RT-Omniscript kit (QIAGEN) according to the manufacturer's instructions.

Quantitative PCR analyses were also perfomed on mRNA obtained using the above described method from peripheral blood mononuclear cells (PBMC) after a blood collection of the same patients.

### 2.2. Results

### PSMB10 expression in healthy renal transplanted patients versus renal transplanted patients suffering from chronic antibody-mediated rejection.

PSMB10 expression was measured by quantitative PCR in peripheral blood mononuclear cells (PBMC) and graft biopsies from renal transplant patients whose graft displays normal histology (N) or signs of chronic antibody-mediated rejection (AMR) (n = 25 for PBMC and n = 43 for biopsies).

Results are displayed in Figure 1A (PBMC) and Figure 1B (renal graft biopsies) and clearly show that the expression level of PSMB10 is significantly increased in renal transplant patients whose graft displays signs of chronic antibody-mediated rejection.

For biopsies, PSMB 10 expression was also measured by quantitative PCR in biopsies displaying interstitial fibrosis and tubular atrophy (IFTA) or calcineurin inhibitor cytotoxicity (CNI tox), which represent lesions distinct from chronic antibody-mediated rejection (AMR). Results are displayed in Figure 2E and show that PSMB10 expression levels are significantly upregulated only in AMR, and not in distinct lesions such as IFTA or CNI tox.

As a result, it appears that PSMB10 expression levels are specifically upregulated in patients with AMR compared to patients with stable graft function or patients with other kinds of graft lesion.

### Sensitivity and specificity of a diagnosis test of chronic graft rejection based on PSMB10 expression level

The sensitivity and specificity of PSMB10 as a diagnosis marker of chronic graft rejection was assessed by Receiver-Operator-Characteristic (ROC) curve analysis of PSMB10 mRNA in the same samples. The ROC is represented as a graphical plot of the sensitivity vs. (1 - specificity) for a binary classifier system as its discrimination threshold is varied. The sensitivity (also referred to as the "true positive fraction") is how good the test is at picking out patients with chronic AMR. Specificity is the ability of the test to pick out patients who do not have chronic AMR. Thus, (1-specificity) is also referred to as the "false positive fraction". The accuracy of the test (i.e. how well the test separates patients with and without chronic AMR) is measured by the Area Under the ROC Curve (AUC) where an AUC of 1.0 represents a perfect test. Thus, for a sensitive and specific diagnosis test, the ROC curve should climb rapidly towards the upper left hand corner of the graph, meaning that the false negative rate is high and the false positive rate is low. Generally, an AUC comprised between 0.7 and 0.8 is considered as acceptable, an AUC comprised between 0.8 and 0.9 is considered as excellent, and an AUC superior to 0.9 is considered as exceptional.

Results obtained for a diagnosis test based on determination of PSMB 10 expression level are displayed in Figure 1C (PBMC) and Figure 1D (renal graft biopsies). The AUC is 1.0 for PSMB10 in the blood and 0.99 for PSMB10 in the biopsies. Such values correspond to an exceptionally sensitive and specific test, much better than tests usually considered as acceptable or even excellent tests.

Another illustration of the discriminative power of a diagnosis test of chronic graft rejection based on PSMB10 expression level is provided in Figure 2, which shows the expression levels of PSMB10 in blood (Figure 2A) or graft biopsy (Figure 2B) for all tested patients, either healthy or undergoing chronic rejection on a horizontal scale, as well as a threshold value of PSMB10 expression level which permits to discriminate the two groups of patients. This graphical representation clearly shows that PSMB10 expression levels of renal transplant patients whose graft displays normal histology segregate under a threshold value both in blood and in graft biopsy, whereas PSMB10 expression levels of renal transplant patients whose graft displays signs of chronic antibody-mediated rejection are significantly higher than the threshold value.

### 2.3. Conclusion

These results clearly demonstrate the potential of PSMB10 for the diagnosis of chronic graft rejection in human transplanted subjects, based on a mere blood collection, or on a biopsy sample.

PSMB10 expression levels may thus be used to diagnose chronic rejection in transplanted subjects. In addition, since the results obtained in blood mirror the results that may be obtained using a biopsy, this marker may be used in a regular control procedure of transplanted subjects, without affecting patients' quality of life or threatening the grafted organ.

### BIBLIOGRAPHY

1. Flechner, S. M., Kurian, S. M., Head, S. R., Sharp, S. M., Whisenant, T. C., Zhang, J., Chismar, J. D., Horvath, S., Mondala, T., Gilmartin, T., Cook, D. J., Kay, S. A., Walker, J. R. & Salomon, D. R. (2004) Am J Transplant 4, 1475-89.
2. WO2005/070086.
3. WO2006/131537.
4. Scherer, A., Krause, A., Walker, J. R., Korn, A., Niese, D. & Raulf, F. (2003) Transplantation 75, 1323-30.
5. Luo H et al. Transplantation. 2001 Jul 27;72(2):196-202.
6. WO 99/22729,
7. WO 2004/014882,
8. WO 2006/099261
9. WO 2005/070086,
10. W02006/131537,
11. WO 2006/099421,
12. Louis S, Braudeau C, Giral M, Dupont A, Moizant F, Robillard N, Moreau A, Soulillou JP, Brouard S. 2006. Contrasting CD25hiCD4+T cells/FOXP3 patterns in chronic rejection and operational drug-free tolerance. 81(3):398-407.
13. Brouard S, Dupont A, Giral M, Louis S, Lair D, Braudeau C, Degauque N, Moizant F, Pallier A, Ruiz C, Guillet M, Laplaud D, Soulillou JP, 2005. Operationally tolerant and minimally immunosuppressed kidney recipients display strongly altered blood T-cell clonal regulation. Am. J. Transplant. 5(2):330-340
14. WO 02/084567.
15. Myung J et al. Med Res Rev. 2001 Jul;21(4):245-73.
16. Guédat P. et al. BMC Biochem. 2007 Nov 22;8 Suppl 1:S14.
17. W02003/033506,
18. W02003/033507,
19. WO2006/086600,
20. WO2005/21558,
21. W02005/016859,
22. WO2004/064755,
23. W02006/017842,
24. WO2003/059898,
25. WO2006/060809,
26. W02005/002572,
27. W02005/099687,
28. WO2004/071382,
29. WO2006/017981.

## Claims

1. A method for diagnosing chronic graft rejection of a grafted organ in a subject from a biological sample of said subject, comprising:
(a) determining in vitro an expression level value for PSMB10 in said subject biological sample,
(b) comparing said value to:
- at least one reference expression level value for PSMB10 in at least one reference sample, or
- a reference threshold value, and
(c) diagnosing if said subject is or not undergoing chronic rejection of said grafted organ.

2. The method according to claim 1, wherein said grafted organ is kidney or heart.

3. The method according to anyone of claims 1-2, wherein said subject biological sample is a blood sample or a grafted organ biopsy.

4. The method according to anyone of claims 1-3, wherein the expression level value for PSMB10 is determined by measuring the amount of nucleic acid transcripts of PSMB10.

5. The method according to claim 4, wherein the expression level value for PSMB10 is determined using quantitative PCR.

6. The method according to anyone of claims 1-5, wherein the obtained expression level value for PSMB10 in said subject biological sample is compared to a reference expression level value for PSMB10 in a corresponding biological sample from a grafted subject with stable graft function.

7. The method according to anyone of claims 1-5, wherein the obtained expression level value for PSMB10 in said subject biological sample is compared both to a reference expression level value for PSMB10 in a corresponding biological sample from a grafted subject with stable graft function and to a reference expression level value for PSMB10 in a corresponding biological sample from a grafted subject who is suffering from chronic rejection.

8. The method according to anyone of claims 1-5, wherein the obtained expression level value for PSMB10 in said subject biological sample is compared to a reference threshold value.

9. The method according to any of claims 1-8, further comprising determining in said subject biological sample the expression level of at least one additional gene.

10. The method according to any of claims 1-9, further comprising determining at least one additional parameter selected from standard biological parameters specific for said subject grafted organ type, phenotypic analyses of peripheral blood mononuclear cells (PBMC), and qualitative and/or quantitative analysis of PBMC immune repertoire.

11. A kit for the diagnosis of a chronic graft rejection, comprising at least one reagent for the determination of PSMB10 expression level in a biological sample, wherein the reagents present in said kit permit to determine the expression level of at most 500 distinct genes.

12. A nucleic acid microarray comprising at least one nucleic acid specific for the PSMB10 gene, wherein said nucleic acid microarray comprises nucleic acids specific for at most 500 distinct genes.

13. A proteasome inhibitor, for use as a medicament for treating chronic graft rejection.

14. The proteasome inhibitor according to claim 9, wherein said proteasome inhibitor is selected from the group consisting of peptide benzamides, peptide α-ketoamides, peptide aldehydes, peptide α-ketoaldehydes, peptide vinyl sulfones, peptide boronic acids, linear peptide epoxyketones, peptide macrocycles, γ-lactam thiol ester, epipolythiodioxopiperazine toxins, salinosporamide A and analogs thereof, (-)-epigallocatechin 3-gallate and analogs thereof.

15. Use of a proteasome inhibitor for preparing a medicament for treating chronic graft rejection.

16. The use according to claim 11, wherein said proteasome inhibitor is selected from the group consisting of peptide benzamides, peptide α-ketoamides, peptide aldehydes, peptide α-ketoaldehydes, peptide vinyl sulfones, peptide boronic acids, linear peptide epoxyketones, peptide macrocycles, γ-lactam thiol ester, epipolythiodioxopiperazine toxins, salinosporamide A and analogs thereof, (-)-epigallocatechin 3-gallate and analogs thereof.
